# EUROPEAN PATENT APPLICATION

(11) **EP 0 808 638 A1**
(43) Date of publication of application: **26.11.1997**
(21) Application number: 97201340.3
(22) Date of filing: 06.05.1997
(51) Int. Cl.: A61M 25/01, A61M 25/06

(54) **Catheter-introduction-sheath with occlusion balloon**

(30) Priority: 20.05.1996 NL 1003172
(71) Applicant: CORDIS EUROPA N.V., NL-9301 LJ Roden (NL)
(72) Inventor: van Erp, Wilhelmus, P.M.M., 9351 KS Leek (NL); Linnewiel, Remco, 9997 PN Yandeweer (NL); Reekers, Jan, A., 1185 CR Amstelveen (NL)
(74) Representative: 't Jong, Bastiaan Jacobus

(57) **Abstract**

The invention relates to a catheter-introduction-sheath comprising a tubular basic body with a proximal and a distal end, a catheter-introduction-lumen extending through the basic body which has been provided with a haemostatic device at the proximal end for the purpose of allowing a catheter to pass through in a sealed manner. At the distal end the basic body carries at least one balloon member which is connected to a supply line for medium under pressure extending from the proximal to the distal end.

## Description

The invention relates to a catheter-introduction-sheath comprising a tubular basic body with a proximal and a distal end. A catheter-introduction-lumen extends through the basic body. When using such a catheter-introduction-sheath it is inserted, through the skin of a patient, into a blood vessel. In that case the introduction sheath forms a guiding means through which a guide wire, catheter et cetera can be introduced into the blood vessel. At the proximal end the basic body has been provided with a haemostatic device which allows a catheter to pass through in a sealed manner, which prevents blood from flowing out of the blood vessel, via the catheter-introduction-lumen, to the outside.

Such a catheter-introduction-sheath is generally known as such and is frequently used.

The catheter-introduction-sheath according to the invention has very advantageous additional application possibilities as the basic body has been provided with at least one balloon member at the distal end, which is connected to a supply lumen for medium under pressure extending from the proximal to the distal end. By supplying a medium under pressure via the supply lumen to the balloon the latter is expanded, as a result of which it can occlude the blood vessel concerned. Consequently the flow of blood in this blood vessel can be stopped.

Thus it is for instance possible to carry out an X-ray investigation of the blood vessel, whereby only a very small quantity of contrast medium needs to be injected. As the contrast medium is not flushed away with the blood it remains for a long period at the required site, so that the surrounding area remains visible on an X-ray screen for a long period of time.

A similar advantageous embodiment of the catheter- introduction-sheath according to the invention is when the latter is used for the purpose of inserting a catheter with which medication is introduced locally. As a result only a relatively small quantity of medication is required to achieve temporarily a high concentration.

Another suitable application of the catheter-introduction-sheath according to the invention is with (hydro-)-thrombectomy-procedures. By stopping the flow of blood in the blood vessel it is prevented that blood thrombi disperse through the vascular system. Thus the risk of an embolism is prevented.

The catheter-introduction-sheath according to the invention can also be used very well for the purpose of removing thrombi by means of a so-called fogoty catheter. The fogoty catheter acts as a kind of scraper with which clots et cetera can be dislodged. The balloon member of the introduction sheath may have been made in such a way that its foremost section forms a funnel pointing in the direction of a lumen in the introduction sheath. The thrombi dislodged by the fogoty catheter are conducted via the balloon into the introduction sheath and are discarded from there.

A very advantageous additional effect of the catheter- introduction-sheath according to the invention is that the occlusion balloon in expanded state centres the distal end of the basic body inside the blood vessel. This is important when the introduction sheath according to the invention is used to introduce catheters comprising a source of radiation for the purpose of treating the tunica intima of the blood vessel. As a result the source of radiation is kept properly at an equal distance from the wall of the blood vessel, so that the radiation treatment will be uniform. Another example of utilizing the centring function is when introducing a guide wire through a thrombus close to the tip of the introduction sheath. As the guide wire is kept properly centred inside the blood vessel, it is prevented that the wall of the blood vessel is damaged when inserting the guide wire through the thrombus whilst using a certain amount of force.

It should be noted that the use of occlusion balloons is known as such. Catheters which have been provided with an occlusion balloon at a distal end are generally known and are used frequently. The catheter-introduction-sheath according to the invention often renders the use of a separate catheter with an occlusion balloon superfluous, so that a considerably greater freedom is obtained when introducing interventional or diagnostic catheters.

With the measure as set out in claim 2 it is achieved, as has been described above, that the distal end of the basic body of the introduction sheath is centred inside the blood vessel when the balloon member is expanded.

A suitable construction of the catheter-introduction-sheath according to the invention has been characterised in claim 3. The channel formed in between the outer and the inner tubular element only needs to have a very small effective cross-section, so that the outer diameter of the basic body remains limited.

Preferably the measure as set out in claim 4 is employed. The supply line for medium under pressure is in that case easily accessible without hindering the introduction of the guide wire and catheter via the haemostatic device.

The invention will be explained in greater detail in the following description with reference to the attached figures.

Figure 1 shows a partly cross-sectional side view of a catheter-introduction-sheath according to the invention.

Figure 2 shows an enlarged detail as indicated by arrow II in figure 1.

Figure 3 shows a perspective view of the catheter-introduction-sheath according to the invention in a position of use.

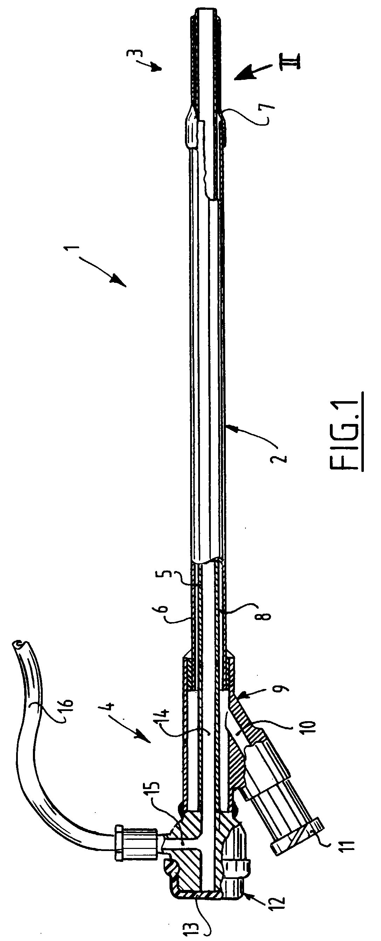

The catheter-introduction-sheath 1 according to the invention illustrated in figure 1 comprises a tubular basic body 2 with a distal end 3 and a proximal end 4.

The basic body 2 has been made up of an inner tubular element 5 and arranged around it an outer tubular element 6.

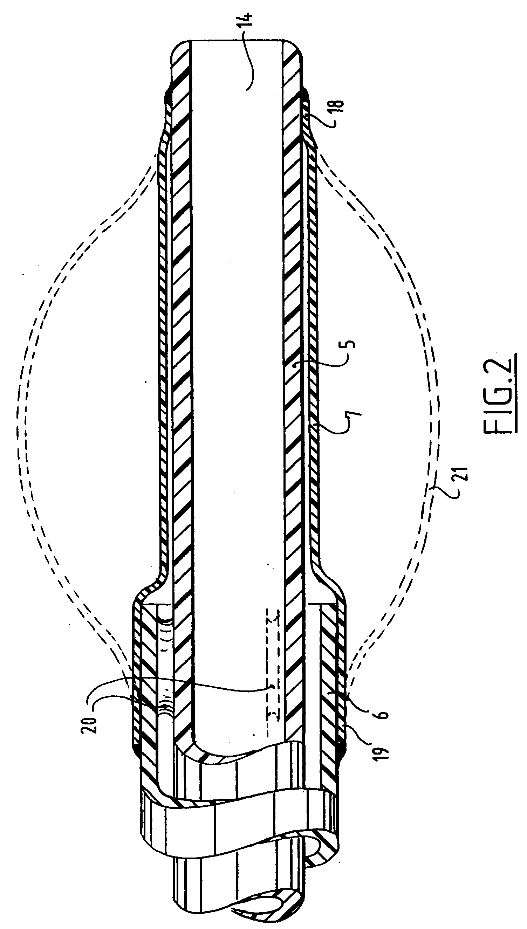

At the distal end 3 the basic body 2 has been provided with a balloon member 7. This balloon member 7 may consist of a piece of tubular element made of a resilient material, for instance latex or may be a preformed balloon member. The balloon member 7 illustrated in the figures has been made of a resilient material, so that in the non-expanded state, illustrated in the figures 1 and 2 by means of continuous lines, it makes close contact with the basic body 2.

According to another embodiment the balloon may also have been made of a non-compliant material and lie, in folded state, against the outer tubular element.

The inner tubular element 5 extends over a distance beyond the distal end of the outer tubular element 6. The relatively distal end 18 of the balloon member 7 is connected to the distal end of the inner tubular element 5, whereas the relatively proximal end 19 of the balloon member 7 is connected to the distal end of the outer tubular element 6. In this way the inside of the balloon member 7 is connected with the channel 8 which is formed in between the inner tubular element 5 and the outer tubular element 6.

As can be seen in figure 2, the inner tubular element 5 may be centred inside the end-section of the outer tubular element by means of connections 20 which are made of a hardened plastic material. The internal diameter of the outer tubular element 6 can only be slightly larger than the external diameter of the internal tubular element 5 however, so that such connections 20 are not absolutely necessary.

The inner tubular element 5 extends into the haemostatic device 12 which has been arranged at the proximal end 4. Consequently a continuous channel 14 is formed inside the inner tubular element 5 for the purpose of advancing a catheter and/or a guide wire. The haemostatic device 12 comprises in the usual manner a membrane 13 which is perforated by the guide wire and/or catheter to be inserted, and forms a seal around this guide wire and/or catheter.

The supply line for medium under pressure formed in between the outer tubular element 6 and the inner tubular element 5 is connected inside the connecting member to a channel 10 inside a side connection 9 of the connecting device. This connection ends in a luer-lock connector 11, so that a line or hypodermic syringe may be connected to it.

Inside the haemostatic device 12, a side channel 15 is also connected to the catheter-introduction-lumen 14. A flushing liquid can be supplied via a line 16. The line 16 can also be used for the connection of for instance a pressure gauge in order to record the pressure inside the blood vessel.

It will be obvious that by supplying medium under pressure via the side channel 10 and the supply line for medium under pressure 8 to the balloon 7, the latter can be transformed from the non-expanded state, drawn with continuous lines in figure 2, into the expanded state 21 drawn with dashed lines.

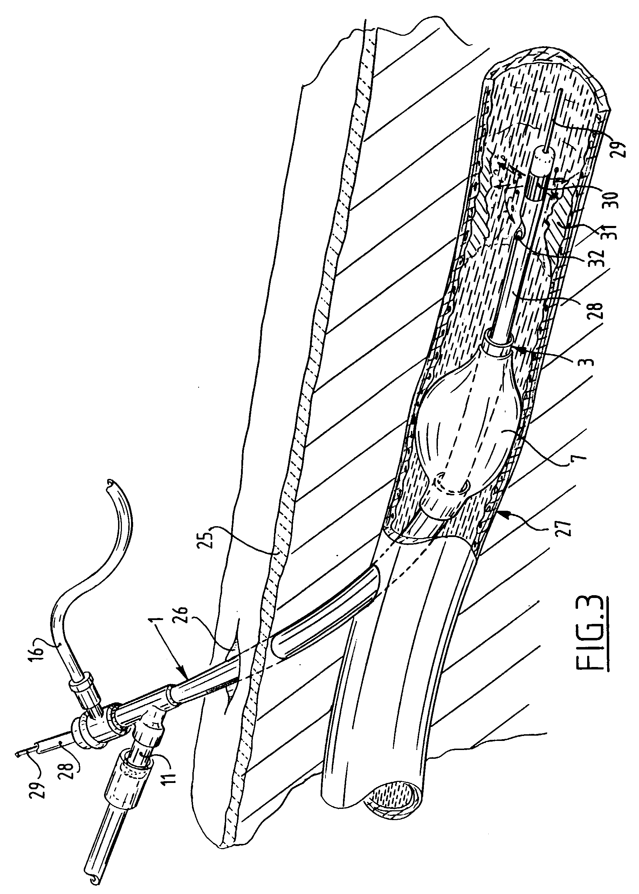

In figure 3 the catheter-introduction-sheath 1 has been illustrated in a position of use with a balloon 7 expanded in the way described above.

The basic body of the catheter-introduction-sheath 1 has been introduced into a blood vessel 27 via an incision 26 in the skin 25 of a patient. A guide wire 29 and a catheter 28 have been introduced into the blood vessel 27 via the catheter-introduction-lumen 14.

As can be seen clearly in figure 3, the distal end 3 of the introduction sheath 1 has been centred in the blood vessel 27 by means of the expanded balloon 7. Consequently the catheter 28 is centred inside the blood vessel 27.

The catheter 28 illustrated in figure 3 is of the type comprising a member emitting radiation 30 which can be used for instance to fragment the thrombi 31 which are stuck to the wall of the blood vessel 27. The fragments of thrombi are discharged via a suction inlet 32 in the catheter 28. The introduction sheath 1 according to the invention ensures on the one hand that the active element 30 of the catheter 28 is centred accurately inside the blood vessel, so that the effective radiation reaches the wall of the blood vessel uniformly, while at the same time the flow in the blood vessel 27 is stopped temporarily as the balloon 7 occludes the blood vessel 27. In this way it is ensured that the dislodged fragments of thrombi are not carried along with the flow of blood but will be discharged in a reliable manner via the suction inlet 32.

## Claims

1. Catheter-introduction-sheath comprising a tubular basic body with a proximal and a distal end, a catheter-introduction-lumen extending through the basic body which has been provided with a haemostatic device at the proximal end for the purpose of allowing a catheter to pass through in a sealed manner, wherein the basic body carries at least one balloon member at the distal end which is connected to a supply line for medium under pressure extending from the proximal to the distal end.

2. Catheter-introduction-sheath as set out in claim 1, wherein the balloon member in expanded state is symmetrical in relation to the centre line of the basic body.

3. Catheter-introduction-sheath as claimed in claim 1 or 2, wherein the basic body comprises an outer tubular element and, received in a central lumen thereof, an inner tubular element, which protrudes at the distal end from the outer tubular element, wherein the balloon member has opposite ends which are connected to the respective distal ends of the tubular elements and the supply line for medium under pressure is formed by a channel formed in between the outer and the inner tubular element.

4. Catheter-introduction-sheath as claimed in one of the previous claims, wherein a two-way connecting member has been arranged at the proximal end comprising a side connection which is connected to the supply line for medium under pressure.
